(19) Europäisches Patentamt
European Patent Office
Office européen des brevets

(11) Veröffentlichungsnummer: **0 026 367**
**B1**

# (12) EUROPÄISCHE PATENTSCHRIFT

(45) Veröffentlichungstag der Patentschrift: **12.05.82**

(51) Int. Cl.³: **C 07 D 265/30**

(21) Anmeldenummer: **80105405.7**

(22) Anmeldetag: **10.09.80**

(54) **Verfahren zur Herstellung von cis-2,6-Dimethylmorpholin.**

(30) Priorität: **25.09.79 DE 2938698**

(43) Veröffentlichungstag der Anmeldung: **08.04.81 Patentblatt 81/14**

(45) Bekanntmachung des Hinweises auf die Patenterteilung: **12.05.82 Patentblatt 82/19**

(84) Benannte Vertragsstaaten: **AT BE CH DE FR GB IT LI NL SE**

(56) Entgegenhaltungen:
**US-A-3 083 202**
**HOUBEN-WEYL**
**»METHODEN DER ORGANISCHEN CHEMIE«, 4. Auflage, Band IV, Teil 2, 1955 Georg Thieme Verlag, Stuttgart, +Seiten 276, 277+**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Himmele, Walter, Dr., Eichenweg 14, D-6909 Walldorf (DE)**
Erfinder: **Hupfer, Leopold, Dr., Waltershoehe 3, D-6701 Friedelsheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von cis-2,6-Dimethylmorpholin

Die Erfindung betrifft ein Verfahren zur Herstellung von cis-2,6-Dimethylmorpholin aus trans-2,6-Dimethylmorpholin durch Isomerisierung an Palladium enthaltenden Katalysatoren in Gegenwart von Wasserstoff.

Es ist bekannt, daß man in einem Gemisch aus cis- und trans-2,6-Dimethylmorpholin durch Erhitzen dieses Gemisches mit konzentrierter oder rauchender Schwefelsäure bei Temperaturen zwischen 185 und 220°C den Anteil der cis-Verbindung erhöhen kann (US-PS 3 083 202). Weiterhin ist bekannt, daß sich in manchen Fällen cis/trans-Umlagerungen an Hydrierkatalysatoren durchführen lassen (Houben-Weyl, »Methoden der organischen Chemie«, Band 4/2, Seiten 227–283). Beispielsweise läßt sich cis-1,4-Dimethylcyclohexan in Gegenwart eines Nickelkatalysators bei 175°C in trans-1,4,-Dimethylcyclohexan umlagern [N. D. Zelinsky, E. J. Margolis, Ber. dtsch. chem. Ges. 65, 1613 (1932)].

Es wurde nun gefunden, daß man cis-2,6-Dimethylmorpholin der Formel I

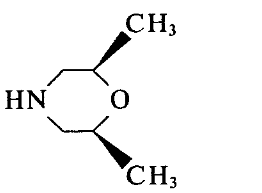

(I)

in einfacher Weise erhält, wenn man trans-2,6-Dimethylmorpholin der Formel II

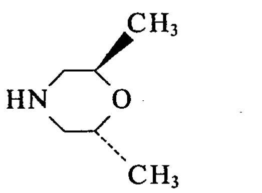

(II)

in Gegenwart von Wasserstoff und einem Palladium enthaltenden Katalysator umsetzt, der neben dem Palladium noch Zink, Cadmium, Mangan oder deren Mischung enthält.

Der Vorteil des erfindungsgemäßen Verfahrens besteht darin, daß man cis-2,6-Dimethylmorpholin weitgehend frei von Neben- und Abfallprodukten erhält. Im Vergleich hierzu führt die bekannte Isomerisierung mit Schwefelsäure bei der anschließenden Neutralisation der Mischung mit Alkalien und Freisetzung der 2,6-Dimethylmorpholinbase zu umweltbelastenden Stoffen in Form von unerwünschten Alkalisulfaten, die den Flüssen zugeleitet werden müssen.

Cis/trans-Umlagerungen des entsprechend substituierten Morpholinringes in Gegenwart von Hydrierkatalysatoren sind bisher nicht bekannt.

Die erfindungsgemäße Umsetzung kann sowohl kontinuierlich als auch diskontinuierlich durchgeführt werden. In der Regel werden Temperaturen zwischen 120 und 280°C, vorteilhaft zwischen 150 und 250°C, und Drücke zwischen 1 (Normaldruck) und 200 bar, vorteilhaft zwischen 1 und 50 bar, angewendet. Es kann lösungsmittelfrei oder in Gegenwart von Lösungsmitteln, die unter den Reaktionsbedingungen inert sind, gearbeitet werden.

Als Lösungsmittel kommen beispielsweise in Frage:

Cyclohexan, Cyclopentan, Methylcyclopentan, Hexan, Heptan, Cyclohexylmethyläther, Di-n-butyläther, Tetrahydrofuran und t-Butanol.

Als Katalysatoren finden Palladiumkatalysatoren Verwendung, die Zink, Cadmium, Mangan oder deren Mischung als Zusatzstoffe enthalten. Diese Katalysatoren werden vorzugsweise mit Trägerstoffen eingesetzt. Als inerte Trägerstoffe eignen sich beispielsweise Aktivkohle, $SiO_2$ oder $Al_2O_3$. Weiterhin wirkt sich ein Zusatz von basischen Metalloxiden, beispielsweise von Oxiden aus der Gruppe der seltenen Erdmetalle, z. B. $Pr_2O_3$, $La_2O_3$, $CeO_2$, $Nd_2O_3$, $Gd_2O_3$ oder $Sm_2O_3$, sehr vorteilhaft aus.

Der Palladiumgehalt des Katalysators, bezogen auf das Trägermaterial, ist nicht kritisch und kann in weiten Grenzen variiert werden. Zweckmäßig ist ein Gehalt von 0,05 bis 15 Gew.-% Palladium. Für die Zusatzkomponenten des Katalysators (Zink, Cadmium, Mangan oder deren Mischung ) ist jeweils ein Gehalt von 0,01 bis 10 Gew.-% – bezogen auf den Träger – zweckmäßig. Das Gewichtsverhältnis der Zusatzkomponenten des Katalysators zu dem Palladiummetall kann beispielsweise zwischen 400 : 1 bis 1 : 150, bevorzugt bei 50 : 1 bis 1 : 10, liegen. Der Gehalt des Katalysators an Oxiden der seltenen Erden kann beispielsweise im Bereich von 0,2 bis 20 Gew.-% liegen. Man kann den Katalysator beispielsweise in Pulverform oder in Form von Strängen verwenden.

Der Katalysator kann in üblicher Weise hergestellt werden, beispielsweise durch Tränkung des inerten Trägerstoffes mit Carbonaten der Metalle und anschließendes Erhitzen.

Das nach dem Verfahren der Erfindung hergestellte cis-2,6-Dimethylmorpholin findet als Zwischenprodukt für die Herstellung von Wirkstoffen in Pflanzenschutzmitteln Verwendung (DE-OS 2 656 747, 2 752 096, 2 752 135).

Die in den folgenden Beispielen genannten Teile bedeuten Gewichtsteile; Raumteile verhalten sich zu ihnen wie Liter zu Kilogramm.

## Beispiel 1

1000 Raumteile eines Wirbelofens mit einem Gesamtvolumen von 1300 Raumteilen werden mit einem Katalysator gefüllt, der aus 0,5 Gew.-% Palladium, 0,11 Gew.-% Zink und 0,1 Gew.-% Cadmium auf Aluminiumoxid besteht.

Der Katalysator wird in Pulverform verwendet und besitzt eine Korngröße von 0,2 bis 0,6 mm. Nach dem Einfüllen wird der Katalysator auf 220°C erhitzt und durch gleichzeitige Zufuhr von 300 000 Raumteilen Wasserstoff pro Stunde und 100 000 Raumteilen Stickstoff pro Stunde in den Wirbelzustand versetzt. Durch diese Katalysator-Wirbelschicht werden stündlich 100 Teile trans-2,6-Dimethylmorpholin geführt. Durch Abkühlung des aus dem Wirbelofen herausströmenden Reaktionsproduktes erhält man pro Stunde 100 Teile Umsetzungsprodukt.

Die gaschromatographische Analyse des Umsetzungsproduktes zeigt einen Gehalt von 86% cis-2,6-Dimethylmorpholin und 14% trans-2,6-Dimethylmorpholin an.

Eine Fraktionierung von 1200 Teilen des Rohrproduktes über eine Füllkörperkolonne mit 60 theoretischen Böden liefert 846 Teile reines cis-2,6-Dimethylmorpholins, $Kp_{100}=80$ bis 81°C. Das entspricht einer Ausbeute von 70,5% (ohne Berücksichtigung des Anteils an trans-2,6-Dimethylmorpholin der in die Wirbelschicht zurückgeführt werden kann). Weiterhin fallen 306 Teile einer Fraktion an, die überwiegend aus trans-2,6-Dimethylmorpholin besteht, $Kp_{100}=87$ bis 90°C. An höhersiedenden Produkten (Destillationsrückstand) werden 48 Teile erhalten. Die Selektivität der Reaktion beträgt 96%.

Beispiel 2

Ein Rührautoklav mit einem Volumen von 300 Raumteilen wird mit einer Mischung aus 150 Teilen trans-2,6-Dimethylmorpholin und 3 Teilen eines Katalysators gefüllt, der aus 10 Gew.-% Palladium, 5 Gew.-% Praseodymoxid und 1 Gew.-% Mangan auf Aluminiumoxid besteht. Der Autoklav wird verschlossen, dann wird Wasserstoff bis zum Erreichen eines Druckes von 15 bar aufgepreßt. Anschließend wird 12 Stunden lang bei 230°C und Eigendruck (ca. 30 bar) erhitzt. Der Reaktionsaustrag wird filtriert und das Filtrat destillativ (ohne Fraktionierung) gereinigt. Das Destlillat, insgesamt 138 Teile, besteht zu 73% aus cis-2,6-Dimethylmorpholin und zu 27% aus trans-2,6-Dimethylmorpholin. Der Destillationsrückstand beträgt 12 Teile. Aus diesen Daten errechnet sich eine Selektivität von 92%.

**Patentansprüche**

1. Verfahren zur Herstellung von cis-2,6-Dimethylmorpholin durch Isomerisierung von trans-2,6-Dimethylmorphclin, dadurch gekennzeichnet, daß man trans-2,6-Dimethylmorpholin in Gegenwart von Wasserstoff und in Gegenwart eines Katalysators, der Palladium und Zink, Cadmium, Mangan oder deren Mischung enthält, bei Temperaturen zwischen 120 und 280°C und Drücken zwischen 1 und 200 bar umsetzt.

2. Verfahren gemäß Anspruch 1, dadurch gekennzeichnet, daß man die Umsetzung bei einer Temperatur zwischen 150 und 250°C durchführt.

3. Verfahren gemäß Anspruch 1, dadurch gekennzeichnet, daß man einen Katalysator verwendet, der Zusätze zwischen 0,2 und 20 Gew.-% eines Oxids oder eines Gemisches von Oxiden der seltenen Erdmetalle enthält.

**Claims**

1. A process for the preparation of cis-2,6-dimethylmorpholine by isomerizing trans-2,6-dimethylmorpholine, characterized in that trans-2,6-dimethylmorpholine is reacted, in the presence of hydrogen and of a catalyst containing palladium als well als zinc, cadmium or manganese or a mixture of these, at from 120 to 280°C unter a pressure of from 1 to 200 bar.

2. A process as claimed in claim 1, characterized in that the reaction is carried out at from 150 to 250°C.

3. A process as claimed in claim 1, characterized in that a catalyst is used which additionally contains from 0.2 to 20% by weight of a rare earth metal oxide or of a mixture of such oxides.

**Revendications**

1. Procédé de préparation de cis-2,6-diméthylmorpholine par isomérisation de trans-2,6-diméthylmorpholine, caractérisé par le fait que l'on fait réagir de la trans-2,6-diméthylmorpholine, en présence d'hydrogène et en présence d'un catalyseur qui contient du palladium et du zinc, du cadmium, du manganèse ou leur mélange, à des températures entre 120 et 280°C et des pressions entre 1 et 200 bars.

2. Procédé selon la revendication 1, caractérisé par le fait que l'on effectue la réaction à une température entre 150 et 250°C.

3. Procédé selon la revendication 1, caractérisé par le fait que l'on utilise un catalyseur qui contient des additions entre 0,2 et 20% en poids, d'un oxyde ou d'un mélange d'oxydes de métaux des terres rares.